# EUROPÄISCHE PATENTSCHRIFT

(11) **EP 1 339 873 B1**
(45) Veröffentlichungstag und Bekanntmachung des Hinweises auf die Patenterteilung: **03.01.2007**
(21) Anmeldenummer: 01982464.8
(22) Anmeldetag: 06.11.2001
(51) Int. Cl.: C12Q 1/68, C12N 15/12

(54) **DIAGNOSE VON MIT HUMOS ASSOZIIERTEN KRANKHEITEN**
DIAGNOSIS OF DISEASES ASSOCIATED WITH THE HUMAN C-MOS GENE
DIAGNOSTIC DE MALADIES ASSOCIEES AU GENE C-MOS HUMAIN

(30) Priorität: 06.11.2000 DE 10054972
(43) Veröffentlichungstag der Anmeldung: 03.09.2003
(73) Patentinhaber: Epigenomics AG, 10178 Berlin (DE)
(72) Erfinder: OLEK, Alexander, 10115 Berlin (DE); PIEPENBROCK, Christian, 10115 Berlin (DE); BERLIN, Kurt, 14532 Stahndorf (DE)
(74) Vertreter: Krauss, Jan
(86) Internationale Anmeldenummer: PCT/EP2001/012831
(87) Internationale Veröffentlichungsnummer: WO 2002/036604

(56) Entgegenhaltungen:
- LING Y-H ET AL: "PACLITAXEL-INDUCED APOPTOSIS IS ASSOCIATED WITH EXPRESSION AND ACTIVATION OF C-MOS GENE PRODUCT IN HUMAN OVARIAN CARCINOMA SKOV3 CELLS" CANCER RESEARCH, AMERICAN ASSOCIATION FOR CANCER RESEARCH, BALTIMORE, MD, US, Bd. 58, Nr. 16, 1998, Seiten 3633-3640, XP000906972 ISSN: 0008-5472
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048
- REIN THEO ET AL: "DNA methylation at mammalian replication origins" JOURNAL OF BIOLOGICAL CHEMISTRY, AMERICAN SOCIETY OF BIOLOGICAL CHEMISTS, BALTIMORE, MD, US, Bd. 274, Nr. 36, 3. September 1999 (1999-09-03), Seiten 25792-25800, XP002182471 ISSN: 0021-9258
- ARAUJO FELIPE D ET AL: "Concurrent replication and methylation at mammalian origins of replication" MOLECULAR AND CELLULAR BIOLOGY, AMERICAN SOCIETY FOR MICROBIOLOGY, WASHINGTON, US, Bd. 18, Nr. 6, Juni 1998 (1998-06), Seiten 3475-3482, XP002182472 ISSN: 0270-7306

## Beschreibung

### Gebiet der Erfindung

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

Die vorliegende Erfindung betrifft Nukleinsäuren und ein Verfahren zur Diagnose von Erkrankungen, die mit dem genetischen und/oder epigenetischen Parametern des Onkogens humos und insbesondere dessen Methylierungsstatus in Zusammenhang stehen.

### Stand der Technik

Das Moloney murine sarcoma virus (MSV) gehört zu den Replikations-inaktiven Retroviren, die Fibroblasten in Kultur transformieren und Sarkome in vivo induzieren. Entstanden ist das Virus durch die Rekombination zwischen dem Moloney murine leukemia virus und einer aus Maus-Zellen entstammenden Sequenz. Das aus Maus-Zellen stammende Segment des MSV, auch als v-mos bezeichnet, wird für die Induktion und Aufrechterhaltung der viralen Transformation benötigt. Homologe Gene zu v-mos sind das ebenfalls aus der Maus stammende c-mos und das humane c-mos (humos), welches dank der evolutionären Konservierung von viralen Onkogenen unter den Vertebratenarten auf dem menschlichen Chromosom 8q11-12 kartiert werden konnte (Prakash K, McBride OW, Swan DC, Devare SG, Tronick SR, Aaronson SA. Molecular cloning and chromosomal mapping of a human locus related to the transforming gene of Moloney murine sarcoma virus. Proc Natl Acad Sci U S A. 1982 Sep;79(17):5210-4; Watson R, Oskarsson M, Vande Woude GF. Human DNA sequence homologous to the transforming gene (mos) of Moloney murine sarcoma virus. Proc Natl Acad Sci U S A. 1982 Jul;79(13):4078-82.). Das c-mos Proto-Onkogen wird bei einer signifikanten Anzahl von Lungenkarzinomen exprimiert und spielt möglicherweise bei deren Entwicklung eine Rolle (Athanasiou A, Gorgoulis VG, Zacharatos P, Mariatos G, Kotsinas A, Liloglou T, Karameris A, Foukas P, Manolis EN, Field JK, Kittas C. c-mos immunoreactivity is an indicator of good prognosis in lung cancer. Histopathology. 2000 Jul;37(1):45-54). Eine Assoziation mit Kehlkopfkrebs wird ebenfalls vermutet (Dolcetti R, Pelucchi S, Maestro R, Rizzo S, Pastore A, Boiocchi M. Proto-oncogene allelic variations in human squamous cell carcinomas of the larynx. Eur Arch Otorhinolaryngol. 1991;248(5):279-85). Die Beteiligung von c-mos an akuter myeloblastischer Leukämie wird dagegen kontrovers diskutiert (Morris CM, Bowen J, Fitzgerald PH. Localization of human c-mos to chromosome band 8q11 in leukemic cells with the t(8;21) (q22;q22). Hum Genet. 1989 Mar;81(4):339-42). Weiterhin wird eine Beteiligung von c-mos an chronischer myelocystischer Leukämie (Huerre C, Despoisse S, Gilgenkrantz S, Lenoir GM, Junien C. c-Ha-rasl is not deleted in aniridia-Wilms' tumour association. Nature. 1983 Oct 13-19;305(5935):638-41) sowie an dem Burkitt's Lymphom vermutet (Klein G. The role of gene dosage and genetic transpositions in carcinogenesis. Nature. 1981 Nov 26;294(5839):313-8).

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek A, Oswald J, Walter J. A modified and improved method for bisulphite based cytosine methylation analysis. Nucleic Acids Res. 1996 Dec 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.
Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z.B. Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO 95/00669) oder einen Enzymschnitt (Xiong Z, Laird PW. COBRA: a sensitive and quantitative DNA methylation assay. Nucleic Acids Res. 1997 Jun 15;25(12):2532-4) nachgewiesen. Zudem ist auch der Nachweis durch Hybridisierung beschrieben worden (Olek et al., WO 99 28498).

Weitere Publikationen, die sich mit der Anwendung der Bisulfit-Technik zum Methylierungsnachweis bei einzelnen Genen befassen, sind: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation pattems in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97 46705, WO 95 15373 und WO 97/45560.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999) und der dort zitierten Literatur entnehmen.

Für die Abtastung eines immobilisierten DNA-Arrays sind vielfach fluoreszenzmarkierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektrometrie eignet sich ausgezeichnet zur Analyse von Peptiden und Proteinen. Die Analyse von Nukleinsäuren ist etwas schwieriger (Gut I G, Beck S. DNA and Matrix Assisted Laser Desorption Ionization Mass Spectrometry. Current Innovations and Future Trends. 1995, 1; 147-57). Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektrometrie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut IG, Beck S. A procedure for selective DNA alkylation and detection by mass spectrometry. Nucleic Acids Res. 1995 Apr 25;23(8): 1367-73). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

### Aufgabenstellung

Die vorliegende Offenbarung beschreibt Oligonukleotide und/oder PNA-Oligomere zur Detektion von Cytosin-Methylierungen und stellt ein Verfahren bereit, welches sich zur Diagnose von genetischen und epigenetischen Parametern des humos-Gens besonders eignet. Der Erfindung liegt die Erkenntnis zugrunde, dass sich insbesondere Cytosin-Methylierungsmuster zur Diagnose von mit humos assoziierten Erkrankungen besonders eignen.

### Beschreibung

Es ist Aufgabe der vorliegenden Erfindung, ein Amplifikat der chemisch modifizierten DNA des Gens humos, sowie ein Verfahren bereit zu stellen, welches sich zur Diagnose von genetischen und epigenetischen Parametern des humos-Gens besonders eignet. Der Erfindung liegt die Erkenntnis zugrunde, dass sich genetische und epigenetische Parameter und insbesondere das Cytosin-Methylierungsmuster des humos-Gens zur Diagnose von mit humos assoziierten Erkrankungen besonders eignen.

Diese Aufgabe wird erfindungsgemäß durch ein Amplifikat gemäß Anspruch 13 gelöst. Die chemisch modifizierte Nukleinsäure konnte bisher nicht in Zusammenhang mit der Ermittlung von genetischen und epigenetische Parametern gebracht werden.

Die vorliegende Offenbarung beschreibt weiterhin ein Oligonukleotid oder Oligomer zur Detektion des Cytosin-Methylierungszustandes in chemisch vorbehandelter DNA, umfassend mindestens eine Basensequenz mit einer Länge von mindestens 13 Nukleotiden, die an eine chemisch vorbehandelte DNA des Gens humos gemäß einer der Seq. ID No.1 bis Seq. ID No.4 hybridisiert. Die Oligomersonden stellen wichtige und effektive Werkzeuge dar, welche die Ermittlung der genetischen und epigenetischen Parameter des humos-Gens erst ermöglichen. Bevorzugterweise umfaßt die Basensequenz der Oligomere mindestens ein CpG Dinukleotid. Die Sonden können auch in Form einer PNA (Peptide Nucleic Acid) vorliegen, die besonders bevorzugte Paarungseigenschaften aufweist. Besonders bevorzugt sind erfindungsgemäße Oligonukleotide, bei denen das Cytosin des CpG Dinukleotids das 5. - 9. Nukleotid vom 5'-Ende des 13 mers ist, im Falle von PNA-Oligomeren ist es bevorzugt, dass das Cytosin des CpG Dinukleotids das 4. - 6. Nukleotid vom 5'-Ende des 9 mers ist.

Die Oligomere werden normalerweise in sogenannten Sets eingesetzt, die für jedes der CpG Dinukleotide eine der Sequenzen der Seq. ID No.1 bis Seq. ID No.4 mindestens ein Oligomer umfassen. Bevorzugt ist ein Set, das für jedes der CpG Dinukleotide aus einer der Seq ID No.1 bis Seq ID No.4 mindestens ein Oligomer umfaßt.

Weiterhin beschreibt die Offenbarung ein Set von mindestens zwei Oligonukleotiden, die als sogenannte Primeroligonukleotide zur Amplifikation von DNA-Sequenzen einer der Seq. ID No.1 bis Seq. ID No.4 oder Abschnitten davon eingesetzt werden können.

Im Falle der Sets von Oligonukleotiden ist es bevorzugt, dass mindestens ein Oligonukleotid an eine Festphase gebunden ist.

Die vorliegende Offenbarung beschreibt weiterhin einen Satz von mindestens 10 Oligomeren (Oligonukleotiden und/oder PNA-Oligomeren), die zur Detektion des Cytosin-Methylierungszustandes in chemisch vorbehandelter genomischer DNA (Seq. ID No.1 bis Seq. ID No.4) dienen. Mit diesen Sonden ist die Diagnose von genetischen und epigenetischen Parametern des humos-Gens möglich. Das Set von Oligomeren kann auch zur Detektion von Single Nucleotide Polymorphismen (SNPs) in der chemisch vorbehandelten DNA des Gens humos gemäß einer der Seq. ID No.1 bis Seq. ID No.4 verwendet werden.

Eine beschriebene Anordnung aus unterschiedlichen Oligonukleotiden und/oder PNA-Oligomeren (ein sogenanntes "Array") ist an eine Festphase gebunden. Dieses Array von unterschiedlichen Oligonukleotid- und/oder PNA-Oligomersequenzen kann dadurch gekennzeichnet sein, dass es auf der Festphase in Form eines rechtwinkligen oder hexagonalen Gitters angeordnet ist. Bevorzugterweise besteht die Festphasenoberfläche aus Silizium, Glas, Polystyrol, Aluminium, Stahl, Eisen, Kupfer, Nickel, Silber oder Gold. Möglich sind jedoch auch Nitrocellulose sowie Kunststoffe wie zum Beispiel Nylon, die in Form von Kugeln oder auch als Harz-Matrizes vorliegen können.

Weiter beschrieben wird ein Verfahren zur Herstellung eines auf einem Trägermaterial fixierten Arrays zur Analyse in Zusammenhang mit humos assoziierten Erkrankungen, bei dem mindestens ein Oligomer an eine feste Phase gekoppelt wird. Verfahren zur Herstellung von solchen Arrays sind zum Beispiel aus der US 5,744,305 mittels Festphasenchemie und photolabilen Schutzgruppen bekannt.

Ein weiterer Gegenstand der Erfindung betrifft die Verwendung eines DNA-Chips zur Analyse in Zusammenhang mit húmos assoziierten Erkrankungen, gemäß Anspruch 16. DNA-Chips sind zum Beispiel aus der US 5,837,832 bekannt.

Beschrieben wird weiter ein Kit, das zum Beispiel aus einer Bisulfit enthaltenden Reagenz, einem Satz von Primeroligonukleotiden umfassend mindestens zwei Oligonukleotide, deren Sequenzen jeweils mindestens einen 18 Basenpaaren langen Abschnitt der im Anhang aufgeführten Basensequenzen (Seq. ID No.1 bis Seq. ID No.4) entsprechen oder zu ihnen komplementär sind, Oligonukleotiden und/oder PNA-Oligomeren sowie einer Anleitung zur Durchführung und Auswertung des beschriebenen Verfahrens bestehen kann. Ein Kit kann jedoch auch nur Teile der vorgenannten Bestandteile enthalten.

Die Erfindung stellt weiterhin ein Verfahren zur Ermittlung von genetischen und/oder epigenetischen Parametern des humos-Gens durch Analyse von Cytosin-Methylierungen und Single Nucleotide Polymorphismen gemäß Anspruch 1 zur Verfügung, das folgende Schritte umfaßt:

In einem ersten Verfahrensschritt wird eine genomische DNA-Probe derart chemisch behandelt, dass an der 5'-Position unmethylierte Cytosinbasen in Uracil, Thymin oder eine andere vom Hybridisierungsverhalten her dem Cytosin unähnliche Base verwandelt werden. Dies wird im folgenden unter chemischer Vorbehandlung verstanden.

Die zu analysierende genomische DNA wird bevorzugt aus den üblichen Quellen für DNA erhalten, wie Zellen oder Zellbestandteilen, zum Beispiel Zelllinien, Biopsine, Blut, Sputum, Stuhl, Urin, Gehim-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger oder Kombinationen davon.

Für die oben beschriebene Behandlung genomischer DNA wird Bisulfit (Hydrogensulfit, Disulfit) und anschließende alkalische Hydrolyse verwendet, die zu einer Umwandlung nicht methylierter Cytosin-Nukleobasen in Uracil oder eine andere vom Basenpaarungsverhalten her dem Cytosin unähnliche Base führt.

Aus dieser chemisch vorbehandelten genomischen DNA werden Fragmente unter Verwendung von Sätzen von erfindungsgemäßen Primeroligonukleotiden und einer bevorzugterweise hitzestabilen Polymerase amplifiziert. Aus statistischen und praktikablen Erwägungen werden bevorzugterweise mehr als zehn unterschiedliche Fragmente amplifiziert, die 100 - 2000 Basenpaare lang sind. Die Amplifikation von mehreren DNA-Abschnitten kann simultan in ein und demselben Reaktionsgefäß durchgeführt werden. Üblicherweise wird die Amplifikation mittels der Polymerasekettenreaktion (PCR) durchgeführt.

Der Satz von Primeroligonukleotiden umfaßt mindestens zwei Oligonukleotide, deren Sequenzen jeweils revers komplementär oder identisch zu einem mindestens 18 Basenpaare langen Abschnitt der im Anhang (Seq. ID No.1 bis Seq. ID No.4) aufgelisteten Basensequenzen sind. Die Primeroligonukleotide sind vorzugsweise dadurch gekennzeichnet, dass sie kein CpG Dinukleotid enthalten.

Erfindungsgemäß bevorzugt ist es, dass bei der Amplifikation mindestens ein Primeroligonukleotid an eine Festphase gebunden ist. Die unterschiedlichen Oligonukleotid und/oder PNA-Oligomersequenzen können auf einer ebenen Festphase in Form eines rechtwinkligen oder hexagonalen Gitters angeordnet sein, wobei die Festphasenoberfläche bevorzugt aus Silizium, Glas, Polystyrol, Aluminium, Stahl, Eisen, Kupfer, Nickel, Silber oder Gold besteht, wobei auch andere Materialien, wie Nitrocellulose oder Kunststoffe verwendet werden können.

Die mittels der Amplifikation erhaltenen Fragmente können eine direkt oder indirekt nachweisbare Markierung tragen. Bevorzugt sind Markierungen in Form von Fluoreszenzmarkierungen, Radionukliden oder ablösbaren Molekülfragmenten mit typischer Masse, die in einem Massenspektrometer nachgewiesen werden können, wobei bevorzugt ist, dass die erzeugten Fragmente zur besseren Detektierbarkeit im Massenspektrometer eine einzelne positive oder negative Nettoladung aufweisen. Der Nachweis kann mittels Matrix assistierter Laser Desorptions/Ionisations Massenspektrometrie (MALDI) oder mittels Elektrospray Massenspektrometrie (ESI) durchgeführt und visualisiert werden.

Die im zweiten Verfahrensschritt erhaltenen Amplifikate werden anschließend an einen Satz von Oligonukleotiden und/oder PNA- Sonden der oder an ein Array hybridisiert. Die Hybridisierung erfolgt dabei auf die unten angegebene Art und Weise. Der bei der Hybridisierung verwendete Satz besteht bevorzugterweise aus mindestens 10 Oligonukleotid oder PNA-Oligomer Sonden. Die Amplifikate dienen dabei als Sonden, die an vorher an einer Festphase gebundene Oligonukleotide hybridisieren. Die nicht hybridisierten Fragmente werden anschließend entfernt. Die besagten Oligonukleotide umfassen mindestens eine Basensequenz mit einer Länge von 13 Nukleotiden, die revers komplementär oder identisch zu einem Abschnitt der im Anhang aufgeführten Basensequenzen ist, der mindestens ein CpG Dinukleotid enthält. Das Cytosin des CpG Dinukleotids ist das 5. bis 9. Nukleotid vom 5'-Ende des 13 mers aus betrachtet. Für jedes CpG Dinukleotid ist ein Oligonukleotid vorhanden. Die besagten PNA-Oligomere umfassen mindestens eine Basensequenz mit einer Länge von 9 Nukleotiden, die revers komplementär oder identisch zu einem Abschnitt der im Anhang aufgeführten Basensequenzen ist, der mindestens ein CpG Dinukleotid enthält. Das Cytosin des CpG Dinukleotids ist das 4. bis 6. Nukleotid vom 5'-Ende des 9 mers aus gesehen. Für jedes CpG Dinukleotid ist ein Oligonukleotid vorhanden.

Im vierten Verfahrensschritt entfernt man die nicht hybridisierten Amplifikate.

Im letzten Verfahrensschritt detektiert man die hybridisierten Amplifikate. Dabei ist bevorzugt, dass an den Amplifikaten angebrachte Markierungen an jeder Position der Festphase, an der sich eine Oligonukleotidsequenz befindet, identifizierbar sind.

Erfindungsgemäss bevorzugt ist es, dass die Markierungen der Amplifikate Fluoreszenzmarkierungen, Radionuklide oder ablösbare Molekülfragmente mit typischer Masse sind, die in einem Massenspektrometer nachgewiesen werden können. Der Nachweis der Amplifikate, Fragmente der Amplifikate oder zu den Amplifikaten komplementäre Sonden im Massenspektrometer ist bevorzugt, wobei man die Detektion mittels Matrix assistierter Laser Desorptions/Ionisations Massenspektrometrie (MALDI) oder mittels Elektrospray Massenspektrometrie (ESI) durchführen und visualisieren kann.

Zur besseren Detektierbarkeit im Massenspektrometer können die erzeugten Fragmente eine einzelne positive oder negative Nettoladung aufweisen. Bevorzugt wird das vorgenannte Verfahren zur Ermittlung von genetischen und/oder epigenetischen Parametern des humos-Gens verwendet.

Die Oligomere oder Arrays derselben werden zur Diagnose einer mit humos assoziierten Krankheit durch Analyse von Methylierungsmustern des humos-Gens verwendet. Erfindungsgemäss bevorzugt ist die Verwendung des Verfahrens zur Diagnose bedeutender genetischer und/oder epigenetischer Parameter innerhalb des humos-Gens.

Auch die Nukleinsäuren der Seq. ID No.1 bis Seq. ID No.4 können für die Diagnose von genetischen und/oder epigenetischen Parametern des humos-Gens verwendet werden.

Die vorliegende Offenbarung beschreibt weiterhin ein Verfahren zur Herstellung eines Diagnostikums zur Diagnose von mit humos assoziierten Krankheiten durch Analyse von Methylierungsmustern des humos-Gens, wobei das Diagnostikum dadurch gekennzeichnet ist, dass mindestens eine Nukleinsäure, gegebenenfalls zusammen mit geeigneten Zusatz- und Hilfsstoffen zu dessen Herstellung verwendet wird.

Die vorliegende Offenbarung beschreibt ein Diagnostikum zur von mit humos assoziierten Krankheiten durch Analyse von Methylierungsmustern des humos-Gens, das mindestens eine Nukleinsäure, gegebenenfalls zusammen mit geeigneten Zusatz- und Hilfsstoffen, umfaßt.

Die vorliegende Offenbarung beschreibt weiterhin die Diagnose und/oder Prognose nachteiliger Ereignisse für Patienten oder Individuen, bei dem die mittels der Erfindung erhaltenen bedeutenden genetischen und/oder epigenetischen Parameter innerhalb des humos-Gens mit einem anderen Satz genetischen und/oder epigenetischen Parameter verglichen werden können und die so erhaltenen Unterschiede als Basis für eine Diagnose und/oder Prognose nachteiliger Ereignisse für Patienten oder Individuen dienen.

Unter dem Begriff "Hybridisierung" im Sinne der vorliegenden Erfindung ist eine Bindung unter Ausbildung einer Duplex-Struktur eines Oligonukleotids an eine vollständig komplementäre Sequenz im Sinne der Watson-Crick Basenpaarungen in der Proben DNA zu verstehen. Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

Mit dem Begriff "funktionelle Varianten" sind alle DNA-Sequenzen bezeichnet, die komplementär zu einer DNA-Sequenz sind, die unter stringenten Bedingungen mit der Referenzsequenz hybridisieren und eine zu dem entsprechenden erfindungsgemäßen Polypeptid ähnliche Aktivität aufweisen.

"Genetische Parameter" im Sinne dieser Erfindung sind Mutationen und Polymorphismen des humos-Gens und zu seiner Regulation weiterhin erforderlicher Sequenzen. Insbesondere sind als Mutationen Insertionen, Deletionen, Punktmutationen, Inversionen und Polymorphismen und besonders bevorzugt SNPs (Single Nucleotide Polymorphisms) zu bezeichnen. Polymorphismen können aber ebenso Insertionen, Deletionen oder Inversionen sein.

"Epigenetische Parameter" im Sinne dieser Erfindung sind insbesondere Cytosin-Methylierungen und weitere chemische Modifikationen von DNA-Basen des humos-Gens und zu seiner Regulation weiterhin erforderliche Sequenzen. Weitere epigenetische Parameter sind beispielsweise die Acetylierung von Histonen, die jedoch mit dem beschriebenen Verfahren nicht direkt analysiert werden kann, sondern wiederum mit der DNA-Methylierung korreliert.

Die Erfindung soll nun im folgenden anhand der Sequenzen, Figuren und Beispiele weiter verdeutlicht werden, ohne daß die Erfindung hierauf eingeschränkt wird.

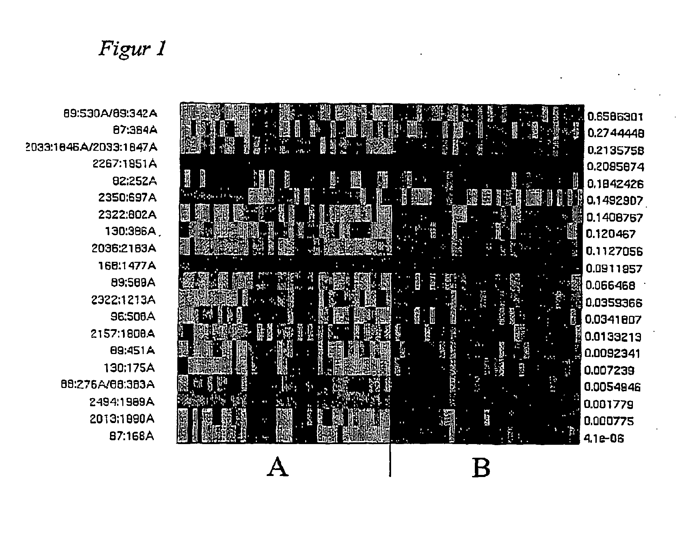

Dabei zeigt Figur 1 die Unterscheidung von Mantel Zell Lymphom (MCL), diffusem großem B-Zell Lymphom (DLBCL) und chronisch lymphatischer Leukämie (CLL/SLL) mit follikulärem Lymphom I (FL I gut differenziert) und follikulärem Lymphom II (FL II mäßig differenziert). Eine hohe Wahrscheinlichkeit für Methylierung entspricht roten Signalen (in der Figur dunkler) eine geringe Wahrscheinlichkeit grüne Signale (in der Figur heller) und schwarz mittleren Werte. Die Proben auf der linken Seite der Figur 1 (A) werden der Gruppe aus MCL, DLBCL und CLL/SLL zugeordnet,die auf der rechten Seite (B) FL I und II (siehe auch Beispiel Nr. 3).

Seq. ID No.1 zeigt die Sequenz der chemisch vorbehandelten genomischen DNA des Gens humos

Seq. ID No.2 zeigt die Sequenz einer zweiten chemisch vorbehandelten genomischen DNA des Gens humos

Seq. ID No.3 zeigt die revers komplementäre Sequenz der Seq. ID 1 der chemisch vorbehandelten genomischen DNA des Gens humos

Seq. ID No.4 zeigt die revers komplementäre Sequenz der Seq. ID 2 der chemisch vorbehandelten genomischen DNA des Gens humos

Seq. ID No.5 zeigt die Sequenz eines Oligonukleotids zur Amplifizierung von humos aus Beispiel 1

Seq. ID No.6 zeigt die Sequenz eines zweiten Oligonukleotids zur Amplifizierung von humos aus Beispiel 1

Seq. ID No.7 zeigt die Sequenz eines Oligonukleotids zur Hybridisierung des Amplifikats von humos aus Beispiel 1

Seq. ID No.8 zeigt die Sequenz eines zweiten Oligonukleotids zur Hybridisierung des Amplifikats von humos aus Beispiel 1

Seq. ID No.9 zeigt die Sequenz eines dritten Oligonukleotids zur Hybridisierung des Amplifikats von humos aus Beispiel 1

Seq. ID No.10 zeigt die Sequenz eines vierten Oligonukleotids zur Hybridisierung des Amplifikats von humos aus Beispiel 1

Seq. ID No.11 zeigt die Sequenz eines Oligonukleotids zur Hybridisierung des Amplifikats von humos aus Beispiel 1

Seq. ID No.12 zeigt die Sequenz eines fünften Oligonukleotids zur Hybridisierung des Amplifikats von humos aus Beispiel 1

Seq. ID No.13 zeigt die Sequenz eines Oligonukleotids zur Hybridisierung des Amplifikats von humos aus Beispiel 1

Seq. ID No.14 zeigt die Sequenz eines sechsten Oligonukleotids zur Hybridisierung des Amplifikats von humos aus Beispiel 1

Seq. ID No.15 zeigt die Sequenz eines siebten Oligonukleotids zur Hybridisierung des Amplifikats von humos aus Beispiel 1

Seq. ID No.16 zeigt die Sequenz eines achten Oligonukleotids zur Hybridisierung des Amplifikats von humos aus Beispiel 1

Seq. ID No.17 zeigt die Sequenz eines achten Oligonukleotids zur Hybridisierung des Amplifikats von humos aus Beispiel 2

Seq. ID No.18 zeigt die Sequenz eines achten Oligonukleotids zur Hybridisierung des Amplifikats von humos aus Beispiel 2

Seq. ID No.19 zeigt die Sequenz eines achten Oligonukleotids zur Hybridisierung des Amplifikats von humos aus Beispiel 2

Seq. ID No.20 zeigt die Sequenz eines achten Oligonukleotids zur Hybridisierung des Amplifikats von humos aus Beispiel 2

Das folgende Beispiel bezieht sich auf ein Fragment des Gens humos, in dem eine bestimmte CG-Position auf ihren Methylierungsstatus hin untersucht wird.

### Beispiel 1: Durchführung der Methylierungsanalyse im humos-Gen

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit im Konzentrationsbereich zwischen 0.1 und 6 M verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen. Im zweiten Verfahrensschritt verdünnt man die behandelte DNA-Probe mit Wasser oder einer wässrigen Lösung. Bevorzugt wird anschliessend eine Desulfonierung der DNA (10-30 min, 90-100 °C) bei alkalischem pH-Wert durchgeführt. Im dritten Schritt des Verfahrens amplifiziert man die DNA-Probe in einer Polymerasekettenreaktion, bevorzugt mit einer hitzebeständigen DNA-Polymerase.

Im vorliegenden Fall werden Cytosine des Gens humos, hier aus der Promotorregion bzw. Exon 1, untersucht. Mit Sequenzen dieses Gens können die Zelllinien MHH-Call2, MHH-Call4, BV-173, 380, NALM-6 und REH (alle vom Typ human B cell precursor leukemia) von CD19+ B-Zellen bzw. CCRF-CEM, Jurkat, Molt-17, P12-Ichikawa (alles human T cell leukemia), R.PMI-8402 (human T cell acute lymphoblastic leukemia) und Karpas-299 (human T cell lymphoma) von CD4+CD8- T-Zellen unterschieden werden. Dazu wird mit den spezifischen Primeroligonukleotiden TTTATTGATTGGGAGTAGGT (Seq. ID 5) und CTAATTTTACAAACATCCTA (Seq. ID 6) ein definiertes Fragment der Länge 494 bp amplifiziert. Dieses Amplifikat dient als Probe, die an ein vorher an einer Festphase gebundenes Oligonukleotid unter Ausbildung einer Duplexstruktur hybridisiert, beispielsweise CCTTACTACGTTAAACTC (Seq. ID 7) oder CCTTACTACATTAAACTC (Seq. ID 8), wobei sich das nachzuweisende Cytosin an Position 164 des Amplifikats befindet. Das methylierte Cytosin wird mit dem Oligonukleotid (Seq. ID 7), welches an der betreffenden komplementären Stelle ein Guanin aufweist, nachgewiesen wogegen die unmethylierte Zustandsform, die durch einThymin repräsentiert wird, mit dem Oligonukleotid (Seq. ID 8), welches an der betreffenden komplementären Stelle ein Adenin aufweist, nachgewiesen wird. Weitere Oligonukleotide, die zur Hybridisierung verwendet werden können beinhalten nachfolgende Sequenzen: GTTACCACCGAACTCCAT (Seq. ID 9) und GTTACCACCAAACTCCAT (Seq. ID 10) mit dem nachzuweisenden Cytosin an Position 263 des Amplifikats, CTCCCCTACGTCCCCCTC (Seq. ID 11) und CTCCCCTACATCCCCCTC (Seq. ID 12) mit dem nachzuweisenden Cytosin an Position 323 des Amplifikats, CTCCAATACGACAATAAA (Seq. ID 13) und CTCCAATACAACAATAAA (Seq. ID 14) mit dem nachzuweisenden Cytosin an Position 89 des Amplifikats, AAACAAACCGTTCACAAC (Seq. ID 15) und AAACAAACCATTCACAAC (Seq. ID 16) mit dem nachzuweisenden Cytosin an Position 401 des Amplifikats.

Der Nachweis des Hybridisierungsprodukts beruht auf Cy5 fluoreszenzmarkierten Primeroligonukleotiden, die für die Amplifikation verwendet wurden. Nur wenn in der Bisulfit behandelten DNA an dieser Stelle ein methyliertes Cytosin vorgelegen hat, kommt es zu einer Hybridisierungsreaktion der amplifizierten DNA mit dem Oligonukleotid. Somit entscheidet der Methylierungsstatus des jeweiligen zu untersuchenden Cytosins über das Hybridisierungsprodukt.

### Beispiel 2: Durchführung der Methylierungsanalyse im humos-Gen

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen. Im zweiten Verfahrensschritt verdünnt man die behandelte DNA-Probe mit Wasser oder einer wässrigen Lösung. Bevorzugt wird anschließend eine Desulfonierung der DNA durchgeführt. Im dritten Schritt des Verfahrens amplifiziert man die DNA-Probe in einer Polymerasekettenreaktion, bevorzugt mit einer hitzebeständigen DNA-Polymerase. Die PCR Reaktionen wurden in einem Thermocycler (Eppendorf GmbH) durchgeführt. Es wurden für einen 25µl Ansatz 10 ng DNA, 0.08µM von jedem Primeroligonukleotid 1,6mM dNTPs und eine Einheit HotstarTaq eingesetzt. Die übrigen Bedingungen wurden gemäß Herstellerangaben gewählt. Für die PCR wurde zuerst eine Denaturierung für 15 Minuten bei 96 °C durchgeführt, danach 46 Zyklen (60 Sekunden bei 96°C, 45 Sekunden bei 55 °C und 75 Sekunden bei 65°C) und eine abschließenden Elongation von 10 Minuten bei 72°C. Das Vorhanden der PCR Produkte wurde auf Agarosegelen überprüft.

Im vorliegenden Fall werden Cytosine des Gens humos untersucht. Mit Sequenzen dieses Gens können Proben von Patienten mit der Diagnose Mantel Zell Lymphom (MCL), diffuses großes B-Zell Lymphom (DLBCL) und chronisch lymphatischer Leukämie (CLL/SLL) von Patienten mit der Diagnose follikuläres Lymphom I (FL I gut differenziert) und follikuläres Lymphom II (FL II mäßig differenziert) unterschieden werden. Dazu wird mit den spezifischen Primeroligonukleotiden TGATTGGGAGTAGGTGTGTT (Seq. ID 17) und CAAATCTTCCAACTTCTCAAA (Seq. ID 18) ein definiertes Fragment der Länge 523 bp amplifiziert. Dieses Amplifikat dient als Probe, die an ein vorher an einer Festphase gebundenes Oligonukleotid unter Ausbildung einer Duplexstruktur hybridisiert, beispielsweise TATGGAGTTCGGTGGTAA (Seq. ID 19) oder TATGGAGTTTGGTGGTAA (Seq. ID 20), wobei sich das nachzuweisende Cytosin an Position 259 des Amplifikats befindet. Das methylierte Cytosin wird mit dem Oligonukleotid (Seq. ID 19), welches an der betreffenden komplementären Stelle ein Guanin aufweist, nachgewiesen, wogegen die unmethylierte Zustandsform, die durch ein Thymin repräsentiert wird, mit dem Oligonukleotid (Seq. ID 20), welches an der betreffenden komplementären Stelle ein Adenin aufweist, nachgewiesen wird. Der Nachweis des Hybridisierungsprodukts beruht auf CY5 fluoreszenzmarkierten Primeroligonukleotiden, die für die Amplifikation verwendet wurden. Nur wenn in der Bisulfit behandelten DNA an dieser Stelle ein methyliertes Cytosin vorgelegen hat, kommt es zu einer Hybridisierungsreaktion der amplifizierten DNA mit dem Oligonukleotid. Somit entscheidet der Methylierungsstatus des jeweiligen zu untersuchenden Cytosins über das Hybridisierungsprodukt.

### Beispiel 3: Digitaler Phänotyp

Das folgende Beispiel beschreibt den Vergleich von Mantel Zell Lymphom (MCL), diffusem großem B-Zell Lymphom (DLBCL) und chronisch lymphatischer Leukämie (CLL/SLL) mit follikulärem Lymphom I (FL I gut differenziert) und follikulärem Lymphom II (FL II mäßig differenziert). Für die Multiplex PCRs wurden wurden fluoreszenzmarkierte Primer benutzt, um 8 Fragmente pro Reaktion zu amplifizieren. Alle PCR Produkte von jedem Individuum wurden gemischt und auf Glassobjektträger hybridisiert, die an jeder Position ein Paar immobilisierte Oligonukleotide trugen. Jedes dieser Detekionsoligonukleotide wurde entworfen, um es gegen bei CpG Stellen befindliche bisulfit konvertierte Sequenzen zu hybridisieren, die entweder im ursprünglichen Zustand unmethyliert (TG) oder methyliert (CG) vorlagen. Die Hybrisierungsbedingungen wurden ausgewählt zur Detektion von Unterschieden bei Einzelnukleotiden der Varianten TG und CG. Die Verhältnisse der beiden Signale wurden basierend auf dem Vergleich der Intensitäten der fluoreszierenden Signale berechnet.

Die Information wird danach in einer gewichteten Matrix (s. Figur 1) bezüglich der CpG Methylierungsunterschieden zwischen zwei Klassen von Geweben bestimmt. Die signifikantesten CpG Positionen werden am unteren Ende der Matrix dargestellt, nach oben hin nimmt die Signifikanz ab. Dunkelgrau (in der Original Figur: rot) zeigt einen hohen Methylierungsgrad an, hellgrau (in der Original Figur: grün) eine niedrigen und schwarz einen mittleren Methylierungsgrad. Jede Reihe repräsentiert eine spezifische CpG Position in einem Gen und jede Spalte zeigt das Methylierungsprofil verschiedener CpGs für eine Probe. Auf der linken Seite werden eine Genidentifizierungsnummer und ein CpG aufgezeigt; der zugehörige Genname ist jeweils in Tabelle 1 zu finden. In Tabelle 1 sind weiterhin die korrespondierenden Accession Nummern der Gene aufgeführt. Die Zahl vor dem Doppelpunkt bezeichnet den Gennamen und die Zahl hinter dem Doppelpunkt das spezifische Oligonukleotid. Auf der rechten Seite von Figur 1 werden die p-Werte der individuellen CpG Positionen gezeigt. Die p-Werte repräsentieren die Wahrscheinlichkeiten, dass die beobachtete Verteilung zufällig auftritt oder nicht.

Die erste (in Figur 1 die linke) Gruppe beinhaltet 42 Proben beiden Geschlechts gegenüber 38 Proben der zweiten (in Figur 1 auf der rechten Seite befindliche) Gruppe. Die p-Wert gewichtete Methylierung zeigt eine klare Unterscheidung zwischen den beiden Gruppen auf, 9 CpG Positionen (rot, bzw. graue Farbschattierung) von 7 verschiedenen Genen werden signifikant unterschieden (korrigierter p-Wert <0.05) zwischen den beiden Gruppen. Die kreuzvalidierte Genauigkeit der Klassifizierung, durch SVM (support vector machine) (F. Model,P. Adorjan,A. Olek,C. Piepenbrock, Feature selection for DNA methylation based cancer classification. Bioinformatics. 2001 Jun;17 Suppl 1:S157-64) bestimmt, wird berechnet als 78,8% mit einer Srtandartabweichung von 2,8%. Im Rahmen einer größeren Studie wurde hier das humos Gen untersucht, welches durch die Gen Identifizierungsnummer 89 dargestellt wird.

**Tabelle 1**

| ***Gen Nummer*** | ***Gen Name*** | ***Accession Nummer*** |
|---|---|---|
| 82 | EGR4 | NM_001965 |
| 87 | AR | NM_000044 |
| 88 | CDK4 | NM_000075 |
| 89 | HUMOS | NM_005372 |
| 96 | RB1 | NM_000321 |
| 130 | GPIbbeta | NM_000407 |
| 168 | MYOD1 | NM_002478 |
| 2013 | BCL2 | NM_000633 |
| 2033 | CDKN1A | NM_000389 |
| 2036 | CDKN2B | NM_004936 |
| 2157 | MLH1 | NM_004936 |
| 2267 | TGFBR2 | NM_003242 |
| 2322 | TP73 | NM_005427 |
| 2350 | CDKN1C | NM_000076 |
| 2494 | BAK1 | NM_001188 |

## Patentansprüche

1. Verfahren zur Ermittlung von genetischen und/oder epigenetischen Parametern geeignet zur Diagnose von bestehenden Erkrankungen oder der Prädisposition für bestimmte Erkrankungen durch Analyse von Cytosin-Methylierungen des humos-Gens, **dadurch gekennzeichnet, dass** man folgende Schritte ausführt:
a) in einer genomischen DNA-Probe werden mittels Behandlung mit einer Lösung eines Bisulfits, Hydrogensulfits oder Disulfits und anschließender alkalischer Hydrolyse an der 5-Position unmethylierte Cytosinbasen in Uracil oder eine andere vom Basenpaarungsverhalten her dem Cytosin unähnliche Base umgewandelt;
b) aus der vorbehandelten DNA werden Fragmente unter Verwendung von Sätzen von mindestens zwei Primeroligonukleotiden umfassend jeweils mindestens eine Basensequenz mit einer Länge von mindestens 9 Nukleotiden, die an eine chemisch vorbehandelte DNA des humos-Gens nach einer der Seq. ID NO 1 bis Seq. ID NO 4 hybridisiert zur Amplifikation von DNA-Sequenzen einer der Seq. ID NO 1 bis Seq. ID NO 4 oder Abschnitten davon und einer Polymerase amplifiziert,
c) die Amplifikate werden an einen Satz von Oligomeren umfassend jeweils mindestens eine Basensequenz mit einer Länge von mindestens 9 Nukleotiden, die an eine chemisch vorbehandelte DNA des humos-Gens nach einer der Seq. ID NO 1 bis Seq. ID NO 4 hybridisiert, oder an eine Anordnung dieser Oligomere; hybridisiert; und
d) die hybridisierten Amplifikate werden anschließend nachgewiesen.

2. Verfahren nach Anspruch 1, **dadurch gekennzeichnet, dass** die Amplifikate eine nachweisbare Markierung tragen.

3. Verfahren nach Anspruch 1 oder 2, **dadurch gekennzeichnet, dass** mehr als zehn unterschiedliche Fragmente amplifiziert werden, die 100 - 2000 Basenpaare lang sind.

4. Verfahren nach einem der Ansprüche 1 bis 3, **dadurch gekennzeichnet, dass** die Amplifikation von mehreren DNA-Abschnitten in einem Reaktionsgefäß durchgeführt wird.

5. Verfahren nach einem der Ansprüche 1 bis 4, **dadurch gekennzeichnet, dass** die Polymerase eine hitzebeständige DNA-Polymerase ist.

6. Verfahren nach einem der Ansprüche 1 bis 5, **dadurch gekennzeichnet, dass** die Markierungen der Amplifikate ausgewählt sind aus Fluoreszenzmarkierungen, Radionukliden oder ablösbaren Molekülfragmenten mit typischer Masse, die in einem Massenspektrometer nachgewiesen werden.

7. Verfahren nach einem der Ansprüche 1 bis 6, **dadurch gekennzeichnet, dass** die Amplifikate oder Fragmente der Amplifikate im Massenspektrometer nachgewiesen werden.

8. Verfahren nach einem der Ansprüche 6 oder 7, **dadurch gekennzeichnet, dass** zur besseren Detektierbarkeit im Massenspektrometer die erzeugten Fragmente eine einzelne positive oder negative Nettoladung aufweisen.

9. Verfahren nach einem der Ansprüche 6 bis 8, **dadurch gekennzeichnet, dass** man die Detektion mittels Matrix assistierter Laser Desorptions/Ionisations Massenspektrometrie (MALDI) oder mittels Elektrospray Massenspektrometrie (ESI) durchführt und visualisiert.

10. Verfahren nach einem der Ansprüche 1 bis 9, **dadurch gekennzeichnet, dass** die genomische DNA aus Zellen oder Zellbestandteilen erhalten wurde, welche DNA enthalten, wobei Quellen von DNA z. B. Zelllinien, Biopsien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

11. Verfahren nach einem der Ansprüche 1 bis 10, **dadurch gekennzeichnet, dass** zwischen der Gruppe von Mantel Zell Lymphom (MCL), diffusem großem B-Zell Lymphom (DLBCL), chronischer lymphatischer Leukämie (CLL/SLL) und der Gruppe von follikulärem Lymphom I (FL I) und follikulärem Lymphom II (FL II) unterschieden wird.

12. Verfahren nach einem der Ansprüche 1 bis 11, wobei die Basensequenz des Oligomers im Schritt c) mindestens ein CpG Dinukleotid umfaßt.

13. Nukleinsäureamplifikat, hergestellt durch
a) Vorbehandlung von an der 5-Position unmethylierten Cytosinbasen in Uracil oder eine andere vom Basenpaarungsverhalten her dem Cytosin unähnliche Base in einer genomischen DNA-Probe mittels Behandlung mit einer Lösung eines Bisulfits, Hydrogensulfits oder Disulfits und anschließender alkalischer Hydrolyse und
b) PCR-Amplifikation von Fragmenten aus der vorbehandelten DNA unter Verwendung von Sätzen von Primeroligonukleotiden umfassend jeweils mindestens eine Basensequenz mit einer Länge von mindestens 18 Nukleotiden, die revers komplementär oder identisch zu einer chemisch vorbehandelten DNA des humos-Gens nach einer der Seq. ID NO 1 bis Seq. ID NO 4 oder Abschnitten davon sind und einer Polymerase, wobei die Fragmente 100 - 2000 Basenpaare lang sind.

14. Amplifikat nach Anspruch 13, das eine nachweisbare Markierung trägt.

15. Amplifikat nach Anspruch 13 oder 14 zur Verwendung in der Diagnose von Erkrankungen.

16. Verwendung eines an eine Festphasenoberfläche gebundenen DNA- und/oder PNA-Arrays eines Satzes von Oligomeren umfassend jeweils mindestens eine Basensequenz mit einer Länge von mindestens 9 Nukleotiden, die identisch ist zu einer chemisch vorbehandelten DNA des humos-Gens nach einer der SEQ ID NO: 1 bis SEQ ID NO: 4 oder Fragmente davon zur Analyse von in Zusammenhang mit dem Methylierungszustand von humos stehenden Erkrankungen.

17. Verwendung eines Arrays nach Anspruch 16 in einem Verfahren zur Unterscheidung zwischen der Gruppe von Mantel Zell Lymphom (MCL), diffusem großem B-Zell Lymphom (DLBCL) chronischer lymphatischer Leukämie (CLL/SLL), und der Gruppe von follikulärem Lymphom I (FL I) und follikulärem Lymphom II (FL II).

## Claims

1. A method for the determination of genetic and/or epigenetic parameters suitable for the diagnosis of existing diseases or the predisposition to specific diseases by analyzing cytosine methylations of the gene humos, **characterized in that** the following steps are carried out:
a) in a genomic DNA-sample, cytosine bases which are unmethylated at the 5-position are converted by chemical treatment by means a solution of a bisulfite, hydrogen sulfite or disulfite, and subsequent alkaline hydrolysis, to uracil or another base which differs from cytosine in terms of its base pairing behavior;
b) fragments from the pretreated DNA are amplified using sets of at least two primer oligonucleotides, each comprising at least one base sequence having a length of at least 9 nucleotides, that hybridize to a chemically pretreated DNA of the gene humos according to one of Seq. ID No. 1 to Seq. ID No. 4 for the amplification of DNA-sequences according to one of Seq. ID No. 1 to Seq. ID No. 4 or segments thereof, and a polymerase,
c) the amplificates are hybridized to a set of oligomers each comprising at least one base sequence having a length of at least 9 nucleotides which hybridizes to a chemically pretreated DNA of the gene humos according to one of Seq. ID No.1 to Seq. ID No.4, or to an array of these oligomers; and
d) the hybridized amplificates are subsequently detected.

2. Method according to claim 1, **characterized in that** the amplificates carry a detectable label.

3. Method according to claim 1 or 2, **characterized in that** more than ten different fragments are amplified, which are 100 - 2000 base pairs in length.

4. Method according to any of claims 1 to 3, **characterized in that** the amplification of several DNA-segments is performed in a single reaction vessel.

5. Method according to any of claims 1 to 4, **characterized in that** the polymerase is a heat-resistant DNA-polymerase.

6. Method according to any of claims 1 to 5, **characterized in that** the labels of the amplificates are selected from fluorescence labels, radionuclides or detachable molecule fragments having a typical mass, which are detected in a mass spectrometer.

7. Method according to any of claims 1 to 6, **characterized in that** the amplificates or fragments of the amplificates are detected in a mass spectrometer.

8. Method according to one of claims 6 or 7, **characterized in that** the generated fragments have a single positive or negative net charge for a better detectability in the mass spectrometer die.

9. Method according to any of claims 6 to 8, **characterized in that** the detection is performed and visualized by means of matrix assisted laser desorption/ionisation mass spectrometry (MALDI) or by means of electrospray mass spectrometry (ESI).

10. Method according to any of claims 1 to 9, **characterized in that** the genomic DNA was obtained from cells or cellular components containing DNA, wherein sources of DNA comprise, e.g., cell lines, biopsies, blood, sputum, stool, urine, brain-spinal cord-fluid, tissue embedded in paraffin, for example tissue of eyes, colon, kidney, brain, heart, prostate, lung, breast or liver, histologic object slides, and all possible combinations thereof.

11. Method according to any of claims 1 to 10, **characterized in that** it is distinguished between the group of mantle cell lymphoma (MCL), diffuse large B-cell lymphoma (DLBCL), chronic lymphatic leukemia (CLL/SLL), and the group of follicular lymphoma I (FL I) and follicular lymphoma II (FL II).

12. Method according to any of claims 1 to 11, wherein the base sequence of the oligomers in step c) comprises at least one CpG dinucleotide.

13. Nucleic acid amplificate, produced by
a) in a genomic DNA-sample, cytosine bases that are unmethylated at the 5-position are converted by means of a pretreatment with a solution of a bisulfite, hydrogen sulfite or disulfite, and subsequent alkaline hydrolysis, into uracil or another base which differs from cytosine in terms of its base pairing behavior;
b) PCR-amplification of fragments from the pretreated DNA using sets of primer oligonucleotides each comprising at least one base sequence having a length of at least 18 nucleotides that are reverse complementary or identically to a chemically pretreated DNA of the gene humos according to one of Seq. ID No. 1 to Seq. ID No. 4 or segments thereof, and a polymerase, wherein the fragments are 100 - 2000 base pairs in length.

14. Amplificate according to claim 13, carrying a detectable label.

15. Amplificate according to claim 13 or 14 for use in the diagnosis of diseases.

16. Use of a DNA- and/or PNA-array of a set of oligomers bound to a solid-phase surface, each comprising at least one base sequence with a length of at least 9 nucleotides, which is identical to a chemically pretreated DNA of the gene humos according to one of SEQ ID No. 1 to SEQ ID No. 4 or fragments thereof for the analysis of diseases that are related to the methylation status of humos.

17. Use of an arrays according to claim 16 in a method for distinguishing between the group of mantle cell lymphoma (MCL), diffuse large B-cell lymphoma (DLBCL) chronic lymphatic leukemia (CLL/SLL), and the group of follicular lymphoma I (FL I) and follicular lymphoma II (FL II).

## Revendications

1. Procédé pour la détermination de paramètres génétiques et/ou épigénétiques, approprié au diagnostic de maladies existantes ou d'une prédisposition à des maladies définies par analyse de méthylations de cytosine de gêne humain, **caractérisé en ce que** les étapes suivantes sont exécutées :
a) dans un échantillon d'ADN génomique, par traitement avec une solution d'un bisulfite, sulfite d'hydrogène ou disulfite et hydrolyse alcaline consécutive en position 5, des bases cytosine non méthylées sont converties en uracile ou en une autre base dont le comportement d'appariement basique est différent de la cytosine ;
b) des fragments d'ADN prétraité sont amplifiés en recourant à des ensembles d'au moins deux oligonucléotides primaires comprenant chacun au moins une séquence de base longue d'au moins 9 nucléotides, hybridée sur un ADN chimiquement prétraité de gêne humain suivant une des séquences ID NO 1 à ID NO 4, pour l'amplification de séquences ADN d'une des séquences ID NO 1 à ID NO 4 ou de tronçons de celle-ci et à une polymérase,
c) les amplificats sont hybridés sur un ensemble d'oligomères comprenant au moins une séquence de base longue d'au moins 9 nucléotides, hybridée sur un ADN chimiquement prétraité de gêne humain suivant une des séquences ID NO 1 à ID NO 4, ou sur un groupement de ces oligomères ; et
d) les amplificats sont ensuite détectés.

2. Procédé selon la revendication 1, **caractérisé en ce que** les amplificats portent un marquage détectable.

3. Procédé selon la revendication 1 ou 2, **caractérisé en ce que** plus de dix fragments différents sont amplifiés, longs de 100 à 2000 paires de base.

4. Procédé selon l'une des revendications 1 à 3, **caractérisé en ce que** l'amplification de plusieurs tronçons d'ADN est exécutée dans un récipient à réaction.

5. Procédé selon l'une des revendications 1 à 4, **caractérisé en ce que** la polymérase est une ADN polymérase résistante à la chaleur.

6. Procédé selon l'une des revendications 1 à 5, **caractérisé en ce que** les marquages des amplificats sont choisis parmi des marquages fluorescents, des radionucléides ou des fragments moléculaires séparables à masse typique, détectés dans un spectromètre de masse.

7. Procédé selon l'une des revendications 1 à 6, **caractérisé en ce que** les amplificats ou des fragments des amplificats sont détectés dans un spectromètre de masse.

8. Procédé selon l'une des revendications 6 ou 7, **caractérisé en ce que** pour une détectabilité améliorée dans le spectromètre de masse, les fragments générés présentent une seule charge nette positive ou négative.

9. Procédé selon l'une des revendications 6 à 8, **caractérisé en ce que** la détection est exécutée et visualisée par spectrométrie de masse à désorption/ionisation laser assurée par matrice (MALDI) ou par spectrométrie de masse à ionisation électrospray (ESI).

10. Procédé selon l'une des revendications 1 à 9, **caractérisé en ce que** l'ADN génomique a été obtenu à partir de cellules ou de composants cellulaires contenant de l'ADN, les sources d'ADN telles que lignes cellulaires, biopsies, sang, expectorations, selles, urine, liquide cérébro-spinal, tissu enrobé de paraffine, p. ex. tissu oculaire, intestinal, rénal, cérébral, cardiaque, prostatique, pulmonaire, mammaire ou hépatique, comprenant des porte-objets histologiques et toutes les combinaisons possibles de ceux-ci.

11. Procédé selon l'une des revendications 1 à 10, **caractérisé en ce qu'**il est distingué entre le groupe de lymphome à cellules du manteau (MCL), de lymphome B diffus à grandes cellules (DLBCL), de leucémie lymphatique chronique (CLL/SLL), et le groupe de lymphome I folliculaire (FL I) et de lymphome II folliculaire (FL II).

12. Procédé selon l'une des revendications 1 à 11, la séquence de base de l'oligomère de l'étape c) comprenant au moins un dinucléotide CpG.

13. Amplificat, produit par
a) prétraitement de bases cytosine non méthylées converties en position 5 en uracile ou en une autre base dont le comportement d'appariement basique est différent de la cytosine dans un échantillon d'ADN génomique, par traitement avec une solution d'un bisulfite, sulfite d'hydrogène ou disulfite et hydrolyse alcaline consécutive, et
b) amplification PCR de fragments d'ADN prétraité en recourant à des ensembles d'oligonucléotides primaires comprenant chacun au moins une séquence de base longue d'au moins 18 nucléotides, inversement complémentaires ou identiques à un ADN chimiquement prétraité de gêne humain suivant une des séquences ID NO 1 à ID NO 4 ou qui sont des tronçons de celle-ci, et à une polymérase, les fragments présentant une longueur de 100 à 2000 paires de base.

14. Amplificat selon la revendication 13, portant un marquage détectable.

15. Amplificat selon la revendication 13 ou 14, utilisable pour le diagnostic de maladies.

16. Utilisation d'un réseau ADN et/ou PNA, lié à une surface de phase solide, d'un ensemble d'oligomères comprenant au moins une séquence de base longue d'au moins 9 nucléotides, identique à un ADN chimiquement prétraité de gêne humain suivant une des séquences ID NO 1 à ID NO 4, ou de fragments de celui-ci pour l'analyse de maladies en relation avec l'état de méthylation du gêne humain.

17. Utilisation d'un réseau selon la revendication 16 dans un procédé de distinction entre le groupe de lymphome à cellules du manteau (MCL), de lymphome B diffus à grandes cellules (DLBCL), de leucémie lymphatique chronique (CLL/SLL), et le groupe de lymphome I folliculaire (FL I) et de lymphome II folliculaire (FL II).


### Sequenzprotokoll

<110> Epigenomics AG
<120> Diagnose von mit humos assoziierten Krankheiten
<150> DE 10054972.1
   <151> 06.11.2000
<160> 20
<210> 1
   <211> 1303
   <212> DNA
   <213> Artificial Sequence
<220>
   <223> chemically treated genomic DNA (Homo sapiens)
<400> 1
   cggaagggaa atgtttttat ttgaaaggga tagttgtgtt ttatttcggt tttttttttt 60
   atttgataaa aatttttgtt gagtgatagt atagatgtag tttatttgga ataagtgaag 120
   gaaaaggaga aaagggatga ggtggagcga aggagtagtt agttatgttt ttaaagtttc 180
   gcggtttttt ttagtttttt tatttatttt agcggttttg gtgttttttt gtaaagtgcg
   240
   atgttttcgt ttttggtttt acgtttttat tttcggagcg agtttttttt atcggtggac
   300
   gcgcggtttt gtagtagttt tttagagtta tttgcgaagt tgtttttggg ggttattttt
   360
   tttcgggttt cgcggttgtc gcgtcggttg gtttggtgtt ttattgattg ggagtaggtg
   420
   tgtttgttgt agaggttggg agttggaggg tttggttcgg tgtataaggc gatttatcgc
   480
   ggtgtttttg tggttataaa gtaagtgaat aagtgtatta agaatcgatt agtatttcgg
   540
   cggagttttt gggttgagtt taacgtagta aggttgcgtt acgataatat cgtgcgcgtg
   600
   gtggttgtta gtacgcgtac gttcgtaggg tttaatagtt tagggattat tattatggag
   660
   ttcggtggta acgttatttt atattaagtt atttatggcg tcgtcggtta ttttgagggg
   720
   gacgtagggg agttttattg tcgtattgga ggatagttaa gtttgggaaa gtgttttaag
   780
   tatttattag atgttgtgaa cggtttgttt tttttttatt cgtaaagtat tgtgtatttg
   840
   gatttgaagt tcgcgaatat tttgattagt gagtaggatg tttgtaaaat tagtgatttc
   900
   ggttgttttg agaagttgga agatttgttg tgtttttaga tattttttta ttttttagga
   960
   ggtatatata tttatcgcgt ttcggagttt ttgaaaggag agggcgtgac gtttaaagtc
   1020
   gatatttatt tttttgttat tattttttgg taaatgatta ttaagtaggc gtcgtattcg
   1080
   ggggagcggt agtatatatt gtacgcggtg gtggtttacg atttgcgttc gttttttttc
   1140
   gttgtcgttt tcgaggattc gtttttcggg tagcgttttg gggacgttat ttagcgttgt
   1200
   tggagattta gcgcggcgta gaggtcgagc gcgcggttgt ttttggtgga ttttattttt
   1260
   ttgaaagttg aattcggttg attgaaaatt tggttaagat aag
   1303
<210> 2
   <211> 1303
   <212> DNA
   <213> Artificial Sequence
<220>
   <223> chemically treated genomic DNA ( Homo sapiens)
<400> 2
   cttatcttaa ccaaattttc aatcaaccga attcaacttt caaaaaaata aaatccacca
         60
   aaaacaaccg cgcgctcgac ctctacgccg cgctaaatct ccaacaacgc taaataacgt
         120
   ccccaaaacg ctacccgaaa aacgaatcct cgaaaacgac aacgaaaaaa aacgaacgca
         180
   aatcgtaaac caccaccgcg tacaatatat actaccgctc ccccgaatac gacgcctact
         240
   taataatcat ttaccaaaaa ataataacaa aaaaataaat atcgacttta aacgtcacgc
         300
   cctctccttt caaaaactcc gaaacgcgat aaatatatat acctcctaaa aaataaaaaa
         360
   atatctaaaa acacaacaaa tcttccaact tctcaaaaca accgaaatca ctaattttac
         420
   aaacatccta ctcactaatc aaaatattcg cgaacttcaa atccaaatac acaatacttt
         480
   acgaataaaa aaaaaacaaa ccgttcacaa catctaataa atacttaaaa cactttccca
         540
   aacttaacta tcctccaata cgacaataaa actcccctac gtccccctca aaataaccga 600
   cgacgccata aataacttaa tataaaataa cgttaccacc gaactccata ataataatcc 660
   ctaaactatt aaaccctacg aacgtacgcg tactaacaac caccacgcgc acgatattat 720
   cgtaacgcaa ccttactacg ttaaactcaa cccaaaaact ccgccgaaat actaatcgat 780
   tcttaataca cttattcact tactttataa ccacaaaaac accgcgataa atcgccttat 840
   acaccgaacc aaaccctcca actcccaacc tctacaacaa acacacctac tcccaatcaa 900
   taaaacacca aaccaaccga cgcgacaacc gcgaaacccg aaaaaaaata acccccaaaa 960
   acaacttcgc aaataactct aaaaaactac tacaaaaccg cgcgtccacc gataaaaaaa 1020
   actcgctccg aaaataaaaa cgtaaaacca aaaacgaaaa catcgcactt tacaaaaaaa 1080
   caccaaaacc gctaaaataa ataaaaaaac taaaaaaaac cgcgaaactt taaaaacata 1140
   actaactact ccttcgctcc acctcatccc ttttctcctt ttccttcact tattccaaat 1200
   aaactacatc tatactatca ctcaacaaaa atttttatca aataaaaaaa aaaaccgaaa 1260
   taaaacacaa ctatcccttt caaataaaaa catttccctt ccg 1303
<210> 3
   <211> 1303
   <212> DNA
   <213> Artificial Sequence
<220>
   <223> chemically treated genomic DNA (Homo sapiens)
<400> 3
   tttattttga ttaagttttt agttagtcga gtttagtttt taaagaggtg agatttatta 60
   aaagtagtcg cgcgttcggt ttttgcgtcg cgttgggttt ttagtagcgt tggatgacgt 120
   ttttaaggcg ttgttcgggg agcgagtttt cgaagacggt agcggagagg gacgggcgta 180
   ggtcgtaggt tattatcgcg tatagtatgt gttgtcgttt tttcgaatac ggcgtttgtt 240
   tggtagttat ttgttagaga gtgatggtaa aggaataaat gtcggtttta ggcgttacgt 300
   tttttttttt taggagtttc ggggcgcggt gggtgtatgt gttttttaga gggtaagagg 360
   gtgtttggaa gtatagtaga ttttttaatt ttttagagta atcgaagtta ttaattttat 420
   agatattttg tttattgatt aagatgttcg cgggttttag gtttaagtgt ataatgtttt 480
   gcgagtggag gaagagtagg tcgtttataa tatttagtga gtatttgaga tattttttta 540
   aatttaattg ttttttagtg cggtagtgag gtttttttgc gtttttttta gggtggtcgg 600
   cggcgttata gatgatttgg tgtaaagtga cgttgttatc gaattttatg atgatggttt 660
   ttaggttatt ggattttgcg ggcgtgcgcg tgttggtagt tattacgcgt acgatgttat 720
   cgtggcgtag ttttgttacg ttgagtttag tttagaaatt tcgtcgagat gttagtcggt 780
   ttttggtgta tttgtttatt tgttttatgg ttataggaat atcgcggtaa gtcgttttgt 840
   atatcgagtt aaatttttta gtttttagtt tttgtagtaa gtatatttgt ttttagttaa
   900
   tggagtatta ggttagtcgg cgcggtagtc gcggggttcg aggaagagtg gtttttagaa
   960
   gtagtttcgt aggtagtttt gagggattgt tgtagggtcg cgcgtttatc gatggggaaa
   1020
   attcgtttcg gaggtagggg cgtagggtta ggggcgaggg tatcgtattt tgtaggggga
   1080
   tattagggtc gttggagtga atgaagagat taggggaaat cgcgggattt tggaaatatg
   1140
   attgattatt ttttcgtttt attttatttt tttttttttt tttttttatt tgttttaaat
   1200
   gagttatatt tgtgttgtta tttagtaaga gtttttatta gatggaggga gaaatcggaa
   1260
   tgaagtatag ttattttttt tagatgaaag tatttttttt tcg
   1303
<210> 4
   <211> 1303
   <212> DNA
   <213> Artificial Sequence
<220>
   <223> chemically treated genomic DNA (Homo sapiens)
<400> 4
   cgaaaaaaaa atactttcat ctaaaaaaaa taactatact tcattccgat ttctccctcc
   60
   atctaataaa aactcttact aaataacaac acaaatataa ctcatttaaa acaaataaaa
   120
   aaaaaaaaaa aaaaaaataa aataaaacga aaaaataatc aatcatattt ccaaaatccc
   180
   gcgatttccc ctaatctctt cattcactcc aacgacccta atatccccct acaaaatacg
   240
   ataccctcgc ccctaaccct acgcccctac ctccgaaacg aattttcccc atcgataaac
   300
   gcgcgaccct acaacaatcc ctcaaaacta cctacgaaac tacttctaaa aaccactctt
   360
   cctcgaaccc cgcgactacc gcgccgacta acctaatact ccattaacta aaaacaaata
   420
   tacttactac aaaaactaaa aactaaaaaa tttaactcga tatacaaaac gacttaccgc
   480
   gatattccta taaccataaa acaaataaac aaatacacca aaaaccgact aacatctcga
   540
   cgaaatttct aaactaaact caacgtaaca aaactacgcc acgataacat cgtacgcgta
   600
   ataactacca acacgcgcac gcccgcaaaa tccaataacc taaaaaccat catcataaaa
   660
   ttcgataaca acgtcacttt acaccaaatc atctataacg ccgccgacca ccctaaaaaa
   720
   aacgcaaaaa aacctcacta ccgcactaaa aaacaattaa atttaaaaaa atatctcaaa
   780
   tactcactaa atattataaa cgacctactc ttcctccact cgcaaaacat tatacactta
   840
   aacctaaaac ccgcgaacat cttaatcaat aaacaaaata tctataaaat taataacttc
   900
   gattactcta aaaaattaaa aaatctacta tacttccaaa caccctctta ccctctaaaa
   960
   aacacataca cccaccgcgc cccgaaactc ctaaaaaaaa aaaacgtaac gcctaaaacc
   1020
   gacatttatt cctttaccat cactctctaa caaataacta ccaaacaaac gccgtattcg
   1080
   aaaaaacgac aacacatact atacgcgata ataacctacg acctacgccc gtccctctcc
   1140
   gctaccgtct tcgaaaactc gctccccgaa caacgcctta aaaacgtcat ccaacgctac
   1200
   taaaaaccca acgcgacgca aaaaccgaac gcgcgactac ttttaataaa tctcacctct
   1260
   ttaaaaacta aactcgacta actaaaaact taatcaaaat aaa
   1303
<210> 5
   <211> 20
   <212> DNA
   <213> Artificial Sequence
<220>
   <223> chemically treated genomic DNA (Homo sapiens)
<400> 5
   tttattgatt gggagtaggt
   20
<210> 6
   <211> 20
   <212> DNA
   <213> Artificial Sequence
<220>
   <223> chemically treated genomic DNA (Homo sapiens)
<400> 6
   ctaattttac aaacatccta
   20
<210> 7
   <211> 18
   <212> DNA
   <213> Artificial Sequence
<220>
   <223> chemically treated genomic DNA (Homo sapiens)
<400> 7
   ccttactacg ttaaactc
   18
<210 > 8
   <211> 18
   <212> DNA
   <213> Artificial Sequence
<220>
   <223> chemically treated genomic DNA ( Homo sapiens)
<400> 8
   ccttactaca ttaaactc                                    18
<210> 9
   <211> 18
   <212> DNA
   <213> Artificial Sequence
<220>
   <223> chemically treated genomic DNA (Homo sapiens)
<400> 9
   gttaccaccg aactccat 18
<210> 10
   <211> 18
   <212> DNA
   <213> Artificial Sequence
<220>
   <223> chemically treated genomic DNA (Homo sapiens)
<400> 10
   gttaccacca aactccat
   18
<210> 11
   <211> 18
   <212> DNA
   <213> Artificial Sequence
<220>
   <223> chemically treated genomic DNA ( Homo sapiens)
<400> 11
   ctcccctacg tccccctc 18
<210> 12
   <211> 18
   <212> DNA
   <213> Artificial Sequence
<220>
   <223> chemically treated genomic DNA (Homo sapiens)
<400> 12
   ctcccctaca tccccctc 18
<210> 13
   <211> 18
   <212> DNA
   <213> Artificial Sequence
<220>
   <223> chemically treated genomic DNA (Homo sapiens)
<400> 13
   ctccaatacg acaataaa 18
<210> 14
   <211> 18
   <212> DNA
   <213> Artificial Sequence
<220>
   <223> chemically treated genomic DNA (Homo sapiens)
<400> 14
   ctccaataca acaataaa 18
<210> 15
   <211> 18
   <212> DNA
   <213> Artificial Sequence
<220>
   <223> chemically treated genomic DNA (Homo sapiens)
<400> 15
   aaacaaaccg ttcacaac 18
<210> 16
   <211> 18
   <212> DNA
   <213> Artificial Sequence
<220>
   <223> chemically treated genomic DNA (Homo sapiens)
<400> 16
   aaacaaacca ttcacaac 18
<210> 17
   <211> 20
   <212> DNA
   <213> Artificial Sequence
<220>
   <223> chemically treated genomic DNA (Homo sapiens)
<400> 17
   tgattgggag taggtgtgtt 20
<210> 18
   <211> 21
   <212> DNA
   <213> Artificial Sequence
<220>
   <223> chemically treated genomic DNA (Homo sapiens)
<900> 18
<210> 19
   <211> 18
   <212 > DNA
   <213> Artificial Sequence
<220>
   <223> chemically treated genomic DNA (Homo sapiens)
<400> 19
   tatggagttc ggtggtaa 18
<210> 20
   <211> 18
   <212> DNA
   <213> Artificial Sequence
<220>
   <223> chemically treated genomic DNA ( Homo sapiens)
<400> 20
   tatggagttt ggtggtaa 18